# EUROPÄISCHE PATENTSCHRIFT

(11) **EP 1 466 600 B1**
(45) Veröffentlichungstag und Bekanntmachung des Hinweises auf die Patenterteilung: **14.05.2008**
(21) Anmeldenummer: 04007309.0
(22) Anmeldetag: 26.03.2004
(51) Int. Cl.: A61K 31/282, A61K 9/08, A61K 47/00, A61P 35/00

(54) **Gebrauchsfertige Oxaliplatin-Lösungen**
Ready-to-use oxaliplatin solutions
Solutions d'oxaliplatine prêtes à l'emploi

(30) Priorität: 28.03.2003 DE 10314377
(43) Veröffentlichungstag der Anmeldung: 13.10.2004
(60) Teilanmeldung: 08004906.7
(73) Patentinhaber: STADA ARZNEIMITTEL AG, 61118 Bad Vilbel (DE)
(72) Erfinder: Schridde, Edgar, 30451 Hannover (DE); Merbach, Bernd, Dr., 30938 Burgwedel (DE); Grimmel, Stefan-Peter, Dr., 30655 Hannover (DE)
(74) Vertreter: Hamm, Volker

(56) Entgegenhaltungen:
- EP-A- 0 943 331
- WO-A-03/004505
- US-A1- 2003 109 514
- US-A1- 2003 109 515

## Beschreibung

Die vorliegende Erfindung betrifft pharmazeutische Zubereitungen von Oxaliplatin (I) zur Verabreichung auf parenteralem Weg. Oxaliplatin (*cis*-Oxalato-(*trans*-1,2-cyclohexandiamin)-platin(II); *cis*-Oxalato-(1,2-cyclohexandiamin)-platin(II), *trans*-1,2-Diaminocyclohexane oxaliplatinum; CAS Nr. 61825-94-3, Mr 397,3; C₈H₁₄N₂O₄Pt) ist eine erstmals im Jahre 2001 im Arzneibuch monographierte Substanz (Pharmeuropa Vol. 13, No 3, 2001, S. 585-588). Sie repräsentiert einen Platin (II)-Komplex mit jeweils einem Äquivalent *trans*-1,2-Diaminocyclohexan und Oxalsäure und hat die chemische Struktur 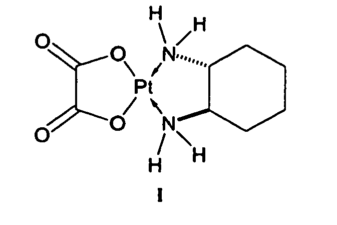 Oxaliplatin ist ein weißes, kristallines Pulver. Es ist löslich in Wasser, dagegen wenig löslich in Methanol und praktisch unlöslich in Ethanol. Es ist anti-neoplastisch wirksam und wird beispielsweise allein, aber auch in Kombination mit 5-Fluorouracil und/oder Folinsäure in der Behandlung des metastasierenden Colorectalcarcinoms therapeutisch eingesetzt. Die empfohlene Dosis in der Oxaliplatin-Therapie beträgt 85 mg/qm Körperoberfläche. Wie andere Platin-Verbindungen kann auch Oxaliplatin zytostatisch bei der therapeutischen Behandlung verschiedenster Krebsarten wie z.B. des Darmkrebses, des Eierstockkrebses oder des Krebses der oberen Atemwege eingesetzt werden. Dabei erfolgt die Verabreichung von Oxaliplatin in der Therapie der verschiedensten Krebserkrankungen intravenös, wodurch es erforderlich wird, den Wirkstoff in Form einer Lösung zur Verfügung zu stellen.

Die aus dem Stand der Technik bekannten, zur parenteralen Verabreichung benötigten Oxaliplatinlösungen werden unmittelbar vor der Verabreichung an den Patienten aus einem Lyophilisat des Oxaliplatins oder aus entsprechenden kristallinem bzw. amorphen Feststoffzubereitungen, die nicht durch Gefriertrocknungsverfahren hergestellt wurden, rekonstituiert. Die Verwendung von solchen Zubereitungen birgt aber wesentliche Nachteile. Zum einen ist das Herstellungsverfahren der Lyophilisate kompliziert und teuer, zum anderen stellt die Rekonstitution zusätzliche Arbeitsschritte dar und bedeutet für das Personal unerwünschte Risiken. Insbesondere kann es bei der Rekonstituierung der Arzneimittellösungen aus einer Trockensubstanz zum sogenannten "Spray-Back-Effect" kommen, durch den es zu einer weiteren Kontamination und Gefährdung des Personals kommen kann. Demgemäss muss sowohl bei der Herstellung des Lyophilisates, als auch bei seiner Rekonstitution jede Kontamination von Personal oder Inventar mit dem hochwirksamen Cytostatikum vermieden werden. Zudem werden an die zur Rekonstitution verwendeten Lösungsmittel hohe Ansprüche gestellt, und es darf sich bei Ihnen nicht um die ansonsten bei Injektionslösungen sehr üblichen Kochsalzlösungen handeln, da es dadurch zu einer Zersetzung des Oxaliplatinkomplexes kommt. Schließlich kann es auch durch andere Fehler in der Handhabung dieser Lyophilisate zu schwerwiegenden Problemen bei der Behandlung mit Oxaliplatin kommen, wie z.B. zu einer Abweichung der Wirkstoffkonzentration oder einer mikrobiellen Kontamination der Lösung. Aufgrund der vielfältigen Gefahren- und Fehlerquellen bei der Benutzung des lyophilisierten Wirkstoffs zur Herstellung von Oxaliplatinlösungen war es daher wünschenswert, gebrauchsfertige, im Folgenden auch ready-to-use-Lösungen genannte, Arzneimittellösungen zur Verfügung zu stellen.

Leider zeigt Oxaliplatin aber in Wasser, wenigstens bei geringen Konzentrationen unterhalb von 1 mg/ml, nur eine sehr geringe Stabilität. In diesem Zusammenhang und der gesamten weiteren Beschreibung der vorliegenden Erfindung ist "Stabilität der Lösung" immer als Stabilität des Oxaliplatin-Komplexes in Lösung zu verstehen, d.h. als langfristige relative Konstanz der Konzentration des Ausgangskomplexes nach Inlösungbringen. Die unzureichende Stabilität von Oxaliplatinlösungen in Wasser resultiert aus der Instabilität des Platinkomplexes selbst, dessen recht labile Liganden durch andere, stärkere bzw. reaktivere Nukleophile ausgetauscht werden können, so dass eine Zerstörung des Ausgangskomplexes resultiert. Deshalb ist die Wahl der Zusätze in Lösungen von Platinkomplexen grundsätzlich kritisch.

Es ist zum Beispiel bekannt, dass Chlorid-Anionen zur Zersetzung von Oxaliplatin führen, was auch der Grund dafür ist, dass Oxaliplatin-Lyophilisate nicht mit Kochsalzlösungen rekonstituiert werden können. Selbst das Hydroxid-Anion aus dem Lösungsmittel Wasser ist in der Lage, Liganden aus einem Platinkomplex zu substituieren, so dass labile Platinkomplexe und dementsprechend auch Oxaliplatin in wässriger Lösung stabilisiert werden müssen. Als durch die Anwesenheit von Hydroxid-Anionen verursachte Zerfalls- bzw. Reaktionsprodukte des Oxaliplatins (Oxalato-DACH-Platin) sind z.B. das Diaquo-DACH-Platin **(II)** und das Diaquo-DACH-Platin-Dimer **(III)** bekannt.

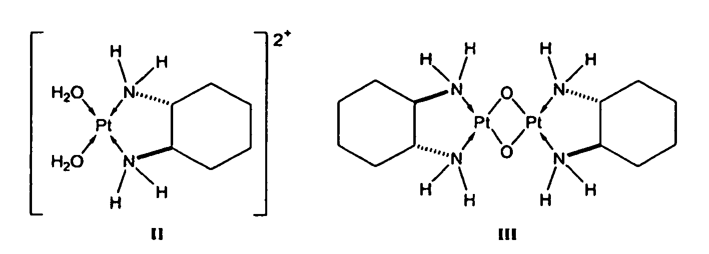

Zur Stabilisierung der Oxaliplatinkomplexe in wässriger Lösung kann die Konzentration bzw. die Aktivität der zersetzenden Anionen, z.B. des Hydroxid-Anions in wässrigen Lösungsmitteln, reduziert werden. Dazu wurde im europäischen Patent EP 0 774 963 vorgeschlagen, die Konzentration des Oxaliplatins auf mindestens 1 mg/ml zu erhöhen, wodurch sich ein saurer pH-Wert zwischen 4,5 und 6 einstellte, die Hydroxid-Anionen Konzentration also erniedrigt ist. Bei Nacharbeitung der Beispiele aus EP 0 774 963 wurden jedoch ausschließlich Lösungen mit pH-Werten größer 6,0 erhalten.

Gemäß EP 0 774 963 sind die darin beschriebenen wässrigen Oxaliplatin-Lösungen ansonsten frei von jedwedem sauren oder alkalischen Mittel, Puffer oder anderen Zusatzstoffen, da deren Interaktion mit dem Oxaliplatin-Komplex und ihre Effekte auf dessen Stabilität nicht vorhersehbar waren.

Nachteilig wirkt sich bei dem in EP 0 774 963 verfolgten Ansatz aus, dass auch die dort erhaltenen Oxaliplatinlösungen nur eine begrenzte Stabilität zeigen. In Vergleichsversuchen (siehe unten) konnte gezeigt werden, dass einfache Oxaliplatinlösungen mit Zusammensetzungen gemäß dem europäischen Patent EP 0 774 963 bereits nach wenigen Stunden erhebliche Konzentrationen der unerwünschten Abbauprodukte **II** und **III** des Oxaliplatins aufweisen. Es besteht somit Bedarf an wässrigen Oxaliplatinlösungen mit weiter verbesserter Stabilität.

Alternativ zur einfachen Konzentrationserhöhung ließe sich die Hydroxid-Anionen-Konzentration grundsätzlich durch Zusatz einer Säure verringern und damit der Platinkomplex in wässriger Lösung stabilisieren. Hierbei besteht jedoch die Gefahr, dass das aus der Säure resultierende Anion zur Zersetzung oder Umbildung des Platinkomplexes führt. Es ist neben der Stabilisierung des Wirkstoffes auch erforderlich, die Bildungsrate sekundärer Abbauprodukte durch Verwendung einer Säure zu verringern. Im Fall von pharmazeutischen *cis*-Platin Lösungen gelingt eine Stabilisierung durch Zusatz von Salzsäure und einer weiteren Chlorid-Ionenquelle, wie z.B. Natriumchlorid. Das entsprechende ChloridAnion führt nicht zu einer Zersetzung des *cis*-Platinkomplexes. Wie oben beschrieben führt derselbe Zusatz aber im Fall des Oxaliplatins zur unerwünschten Zerstörung des Komplexes durch Substitution der labilen Liganden durch Chlorid-Ionen.

Äquivalent zum Zusatz von Salzsäure und Kochsalz zu einer wässrigen *cis*-Platinlösung, also der Verwendung der Säure eines Komplexliganden und des Komplexliganden in freier Form (entsprend einem gleichionigen Zusatz), zur Stabilisierung des Platinkomplexes, wurde im europäischen Patent EP 0 943 331 gezeigt, dass die Zugabe von Oxalat-Anionen und Oxalsäure, also eines Oxalat-Puffers, die Stabilität von Oxaliplatinlösungen in Wasser stark erhöhen kann. Zu der durch die Säure bewirkten Stabilisierung des Oxaliplatins durch Erniedrigung der Hydroxid-Anionen-Konzentration kommt im Fall des gleichionigen Zusatzes ein weiterer stabilisierender Effekt hinzu. Durch den Zusatz eines Liganden des Ausgangskomplexes als freie Spezies, also des Chlorid- oder des Oxalat-Anions, wird die Stabilität von *cis-* bzw. Oxaliplatin weiter erhöht.

In EP 0 943 331 wurde zudem gezeigt, dass weder Citronensäure, noch Essigsäure oder die Aminosäure Glycin geeignet sind, den Oxaliplatinkomplex in wässriger Lösung zu stabilisieren. Die entsprechenden Anionen wirken sich offensichtlich nachteilig auf die Komplexstabilität des Oxaliplatins aus. Zudem wird in diesem Dokument beschrieben, dass das Phosphatanion unter neutralen Bedingungen Oxaliplatin enthaltende wässrige Arzneimittellösungen ebenfalls nicht stabilisieren kann. Vielmehr wurde gezeigt, dass diese Anionen bei den gewählten pH-Werten ungeeignete, hochreaktive und den Oxaliplatinkomplex zersetzende Spezies darstellen.

Leider birgt auch die in EP 0 943 331 vorgeschlagene Stabilisierung von Oxaliplatin in wässriger Lösung erhebliche Nachteile. Weder Oxalsäure noch Oxalat sind zum gegenwärtigen Zeitpunkt als pharmazeutisch geeignete Hilfsstoffe in Arzneibüchern monographiert.

Beide bilden mit Calcium- und Magnesiumkationen, die Bestandteil des Blutes sind, wasserunlösliche Salzkristalle. Insbesondere in der intravenösen Therapie bergen höhere Konzentrationen Oxalsäure und Oxalate das Risiko lokaler und systemischer Nebenwirkungen (lokaler Injektionsschmerz, Thrombozytenaggregation, Thrombose, Nierensteine), so dass die Gabe von Oxalaten als Bestandteil von Injektabilia im Allgemeinen unerwünscht ist.

Um diesen Nachteil zu vermeiden, ist es erforderlich und wesentliche Aufgabe der vorliegenden Erfindung, einen pharmazeutisch geeigneteren und physiologisch verträglicheren Hilfsstoff zu finden, der in gleicher oder besserer Weise geeignet ist, den pH-Wert der Oxaliplatinlösung auf einen physiologisch verträglichen und den Oxaliplatinkomplex stabilisierenden pH-Wert einzustellen, ohne dass das begleitende Säureanion den Oxaliplatinkomplex nachteilig in seiner Stabilität beeinträchtigt. Aus dem gegenwärtigen Stand der Technik und der Lehre zur Stabilität von Oxaliplatin in wässriger Lösung bestehen keine Hinweise auf derartig geeignete Säuren. Insbesondere existieren keine Hinweise auf geeignete Säureanionen.

Aufgabe der vorliegenden Erfindung ist es damit, stabile, gebrauchsfertige Oxaliplatinlösungen zur Verfügung zu stellen, die nicht die zuvor diskutierten Nachteile der bekannten Lösungen aufweisen. Eine weitere Aufgabe dieser Erfindung ist es, Verfahren zur Herstellung solcher Lösungen anzugeben.

Gerade im Hinblick darauf, dass EP 0 943 331 von der Verwendung von Phosphat-enthaltenden Lösungen weglehrt, war es überraschend, dass in den Experimenten zu vorliegender Erfindung gefunden wurde, dass Säuren, deren korrespondierende Anionen sich durch eine geringe Nukleophilie auszeichnen, sowie entsprechenden Puffersysteme in der Lage sind, Oxaliplatin in wässriger Lösung selbst bei einer Konzentration bis hinunter zu 0,025 mg/ml zu stabilisieren.

Als geeignete erfindungsgemäße Säuren, deren Anion die Stabilität des Platinkomplexes und der Lösung nicht beeinträchtigt, sind insbesondere die Oxosäuren der Nichtmetalle Phosphorsäure und Schwefelsäure zu nennen. Des weiteren können die pH-Werte der Oxaliplatinlösungen durch Puffer bestehend aus den oben genannten Säuren sowie deren wasserlöslichen Alkali- und Erdalkalimetallsalzen eingestellt werden.

In den der Erfindung zugrundeliegenden Untersuchungen konnte gezeigt werden, dass Phosphorsäure und Schwefelsäure geeignete Säuren zur pH-Werteinstellung und Stabilitätsverbesserung einer Oxaliplatinlösung sind, und dass deren Anionen gegenüber dem Oxaliplatinkomplex derart unreaktiv sind, dass eine Zersetzung des Oxaliplatinkomplexes weitgehend vermieden wird, wenn der pH-Wert einer Oxaliplatinlösung mit Phosphorsäure oder auch Schwefelsäure eingestellt wird.

Die erfindungsgemäßen Lösungen umfassen bevorzugt zwischen 0,025 mg und 20 mg Oxaliplatin pro ml Wasser. Erfindungsgemäß werden die Lösungen durch den Zusatz einer Säure, deren Anion die Lösung nicht destabilisiert, auf einen pH-Wert von 3 bis 6 eingestellt; besonders bevorzugt wird ein pH-Wert von 3,5 bis 5,0 eingestellt.

Die erfindungsgemäßen Oxaliplatinlösungen können neben den vorgenannten Säuren oder Kombinationen davon auch beliebige pharmazeutisch akzeptable Zusätze enthalten. Beispielhaft können diese Zusätze Kohlenhydrate wie etwa Lactose, Glukose, Maltose, Fruktose, Galaktose, Saccharose und/oder Dextrane umfassen.

Der Zusatz der vorgenannten Kohlenhydrate zu den erfindungsgemäßen Lösungen ermöglicht auch höhere Konzentrationen an Oxaliplatin, ohne dass es zu einer unerwünschten Ausfällung bzw. Auskristallisation des Wirkstoffs kommt. Bevorzugt werden auch Lösungen mit einem Gehalt an einem Kohlenhydrat durch Zusatz einer Säure stabilisiert. Gerade bei Wirkstoffkonzentrationen von mehr als 5 mg/ml ist der Zusatz eines Kohlenhydrats als Löslichkeitsverbesserer von Vorteil. Gemäß einer besonders bevorzugten Ausführungsform umfasst die erfindungsgemäße Lösung 5 mg/ml Oxaliplatin, 55 mg/ml Glukose-Monohydrat und Phosphorsäure in einer Menge, um einen pH-Wert von ca. 3,5 einzustellen.

In den nachfolgenden Beispielen wird die Eignung der erfindungsgemäßen Säuren insbesondere deren Anionen (Phosphat, Sulfat) im Vergleich zur Oxalsäure (Anion: Oxalat) sowie bevorzugte Ausführungsformen der Erfindung veranschaulicht.

### Beispiel 1 - Vergleichsversuche zur Stabilität von wässrigen Oxaliplatinlösungen ohne weitere Zusätze gemäß EP 0 774 963.

Zur Bestimmung der Stabilität von unstabilisierten Oxaliplatinlösungen, wie sie aus EP 0 774 963 bekannt sind wurden Oxaliplatin-Lösungen (C = 2,0 mg/ml, mit Wasser für Injektionszwecke als Lösungsmittel) unter Lichtausschluss bei Temperaturen zwischen 20 und 25°C gelagert, und die relativen Konzentrationen der Oxaliplatin-Zersetzungsprodukte Diaquo-DACH-Platin **(II)** und Diaquo-DACH-Platin Dimer **(III)** mittels HPLC zeitaufgelöst bestimmt. Aus den Mengen der platinhaltigen Zersetzungsprodukte ließ sich der Gehalt des Zersetzungsprodukts Oxalsäure berechnen. Die Ergebnisse dieser Untersuchungen sind in Tabelle 1 zusammengefasst.

**Tabelle 1: Stabilitäten von Oxaliplatin-Lösungen (C = 2,0 mg/ml) ohne weiteren Zusatz. Der pH-Wert ergibt sich zu 6,6; Lagertemperatur 20 - 25 °C.**

| Dauer (h) | Diaquo-DACH-Platin **(II)^{a}** | Diaquo-DACH-Platin Dimer **(III)^{a}** | Oxalsäure^{b} |
|---|---|---|---|
| | (% w/w) | (% w/w) | (% w/w) |
| 0 | 0,047 | - ^{c} | 0,012 |
| ½ | 0,130 | - ^{c} | 0,034 |
| 1 | 0,210 | - ^{c} | 0,056 |
| 2 | 0,335 | - ^{c} | 0,089 |
| 18 | 0,890 | 0,349 | 0,329 |

| | | | |
|---|---|---|---|
| a) relativ zu Oxaliplatin; Gehalt bestimmt mittels HPLC. b) relativ zu Oxaliplatin; Gehalt berechnet aus den Anteilen der Zerfallsprodukte. c) unterhalb der Nachweisgrenze von 0.03% (w/w). | | | |

Wie der Tabelle 1 zu entnehmen ist, ist die Konzentration der Oxaliplatin-Zersetzungsprodukte stetig ansteigend, und der Gehalt an Diaquo-DACH-Platin (II) überschreitet bereits nach 1 h den Wert von 0,2%. Bereits nach 18 h wurde eine erhebliche Bildung des toxikologisch besonders relevanten Diaquo-DACH-Platin Dimers **(III)** festgestellt. Damit wird aus den in Tabelle 1 zusammengefassten Experimenten deutlich, dass Oxaliplatin-Zusammensetzungen ohne stabilisierende Zusätze, wie sie im europäischen Patent EP 0 774 963 beschrieben werden, keine ausreichende Stabilität zeigen, um lagerfähig und über einen längeren Zeitraum pharmazeutisch verwendbar zu sein.

Zur Herstellung lagerfähiger Oxaliplatin-Zusammensetzungen, die über einen längeren Zeitraum eine pharmakologisch akzeptable Stabilität zeigen, besteht also Bedarf an stabilisierenden Zusätzen. Mit der vorliegenden Erfindung ist es gelungen, solche zu identifizieren.

### Beispiel 2 - Vergleichsversuche zur Stabilität von wässrigen Oxaliplatinlösungen stabilisiert durch Zusatz von Oxalsäure gemäß EP 0 943 331, Phosphorsäure oder Schwefelsäure.

Zum Vergleich der Qualität der erfindungsgemäßen Stabilisierung von Oxaliplatinlösungen durch andere Säuren als Oxalsäure, wurden die durch Zugabe von Phosphorsäure und Schwefelsäure erzielten Stabilitäten mit der verglichen, die durch einen aus dem Stand der Technik bekannten gleichionigen Zusatz (Oxalsäure) erreicht wird. Aus EP 0 943 331 war bekannt, dass durch Zusatz von Oxalsäure stabile, wässrige Oxaliplatinlösungen (mit den bekannten Nachteilen) erhalten werden können. Die im folgenden zusammengefassten Ergebnisse verdeutlichen die durch den erfindungsgemäßen Säurezusatz erzielte Verbesserung der Oxaliplatinlösungsstabilitat.

### Versuchsdesign der Experimente unter beschleunigten Bedingungen

Mit Hilfe von bei 20 - 25 °C hergestellten und bei 60 °C gelagerten Versuchschargen, in denen die Konzentration des Oxaliplatins in allen Versuchsreihen C = 6,6 mg/ml betrug, wurde der Einfluss des pH-Wertes und der Wahl der Säure hinsichtlich einer effektiven Stabilisierung geprüft, wobei die pH-Werte jeweils mit 1 N Phosphorsäure bzw. 1 N Schwefelsäure im Vergleich zu 1 N Oxalsäure eingestellt wurden. Dazu wurden jeweils 33 mg Oxaliplatin in 5,0 ml pH-voreingestelltem Lösungsmittel (Wasser für Injektionszwecke, dessen pH-Wert zuvor mit 1 N Phosphorsäure, 1 N Schwefelsäure oder 1 N Oxalsäure eingestellt wurde) auf einem Schüttler innerhalb von 16 min sichtbar klar gelöst und unverzüglich einer HPLC-Analyse zur Ermittlung des Startwerts zugeführt. Die erhaltenen Testansätze wurden nach unverzüglicher Durchführung der ersten Reinheitsananlyse (innerhalb von 20 min nach Klarlösung) bei 60 °C unter Lichtausschluss, unter "beschleunigten Bedingungen" inkubiert und zu den vorgesehenen Prüftakten (nach insgesamt 6 Stunden (h) sowie nach insgesamt 7 Tagen (d)) weiteren Analysen unterzogen. Die pH-Wertbestimmungen erfolgten in allen Messungen potentiometrisch. Die bei diesen Versuchen erhaltenen Ergebnisse sind in den Tabellen 2 und 3 zusammengefasst.

**Tabelle 2: Stabilitäten von wässrigen Oxaliplatinlösungen stabilisiert durch Phosphorsäure bzw. Schwefelsäure und Vergleich mit Lösungen umfassend Oxalsäure (unter beschleunigten Bedingungen, T = 60°C).**

| pH-Wert | Lagerung | mit Oxalsäure | | mit Phosphorsäure | | mit Schwefelsäure | |
|---|---|---|---|---|---|---|---|
| | | **II** | **III** | **II** | **III** | **II** | **III** |
| | | (% w/w) | (% w/w) | (% w/w) | (% w/w) | (% w/w) | (% w/w) |
| 2,0 | Start, 20-25°C | 0,057 | <0,01 | 0,080 | 0,000 | 0,068 | <0,01 |
| | 6 h, 60°C | 0,125 | <0,01 | 1,900 | 0,000 | 2,067 | <0,01 |
| | 7 d, 60°C | 0,135 | <0,01 | 2,36 | <0,01 | 2,540 | <0,01 |
| 2,5 | Start, 20-25°C | 0,045 | <0,01 | 0,055 | <0,01 | 0,042 | <0,01 |
| | 6 h, 60°C | 0,106 | <0,01 | 1,110 | <0,01 | 1,117 | <0,01 |
| | 7 d, 60°C | 0,110 | <0,01 | 1,410 | <0,01 | 1,470 | <0,01 |
| 3,0 | Start, 20-25°C | 0,016 | <0,01 | 0,068 | <0,01 | 0,025 | <0,01 |
| | 6 h, 60°C | 0,085 | <0,01 | 0,653 | <0,01 | 0,589 | <0,01 |
| | 7 d, 60°C | 0,090 | <0,01 | 0,670 | <0,01 | 0,64 | <0,01 |
| 3,5 | Start, 20-25°C | 0,006 | <0,01 | 0,010 | <0,01 | <0,01 | <0,01 |
| | 6 h, 60°C | 0,034 | <0,01 | 0,154 | <0,01 | 0,147 | 0,018 |
| | 7 d, 60°C | 0,090 | <0,01 | 0,260 | 0,010 | 0,220 | 0,010 |
| 4,0 | Start, 20-25°C | <0,01 | <0,01 | <0,01 | <0,01 | 0,010 | <0,01 |
| | 6 h, 60°C | 0,062 | 0,066 | 0,082 | 0,040 | 0,088 | 0,040 |
| | 7 d, 60°C | 0.013 | 0,020 | 0,160 | 0,101 | 0,170 | 0,120 |
| 4,5 | Start, 20-25°C | 0,007 | <0,01 | 0,010 | <0,01 | 0,011 | <0,01 |
| | 6 h, 60°C | 0,084 | 0,140 | 0,086 | 0,219 | 0,091 | 0,090 |
| | 7 d, 60°C | 0,200 | 0,222 | 0,190 | 0,100 | 0,190 | 0,258 |
| 5,0 | Start, 20-25°C | 0,013 | <0,01 | <0,01 | <0,01 | 0,013 | <0,01 |
| | 6 h, 60°C | 0,168 | 0,071 | 0,163 | 0,082 | 0,189 | 0,070 |
| | 7 d, 60°C | 0,190 | 0,090 | 0,190 | 0,090 | 0,180 | 0,090 |
| 5,5 | Start, 20-25°C | 0,012 | <0,01 | 0,027 | <0,01 | <0,01 | <0,01 |
| | 6 h, 60°C | 0,182 | 0,096 | 0,171 | 0,074 | 0,180 | 0,090 |
| | 7 d, 60°C | 0,190 | 0,100 | 0,180 | 0,100 | 0,200 | 0,130 |
| 6,0 | Start, 20-25°C | <0,01 | <0,01 | 0,022 | <0,01 | 0,021 | <0,01 |
| | 6 h, 60°C | 0,157 | 0,124 | 0,170 | 0,076 | 0,162 | 0,102 |
| | 7 d, 60°C | 0,190 | 0,110 | 0,190 | 0,110 | 0,190 | 0,120 |

**Tabelle 3: Zusammenfassung der Stabilitätsmessungen unter beschleunigten**

| | | | | | | | | | |
|---|---|---|---|---|---|---|---|---|---|
| Bedingungen (7 d, T = 60°C;siehe Tabelle 2). | | | | | | | | | |
| A) %w/w Oxalsäure | | | | | | | | | |
| B) %w/w **II;** %w/w **III;** %w/w Summe unbekannter Verbindungen | | | | | | | | | |
| C) %w/w Summe aller Pt-enthaltenden Verunreinigungen | | | | | | | | | |

| pH-Wert | mit Oxalsäure | | | mit Phosphorsäure | | | mit Schwefelsäure | | |
|---|---|---|---|---|---|---|---|---|---|
| | A) | B) | C) | A) | B) | C) | A) | B) | C) |
| | | 0,14 | | | 2,36 | | | 2,54 | |
| 2,0 | 72,42 | <0,01 | 0,62 | 4,27 | <0,01 | 3,09 | 4,23 | <0,01 | 2,86 |
| | | 0,48 | | | 0,73 | | | 0,32 | |
| | | 0,11 | | | 1,41 | | | 1,47 | |
| 2,5 | 21,94 | <0,01 | 0,36 | 1,92 | <0,01 | 1,61 | 1,89 | <0,01 | 1,61 |
| | | 0,25 | | | 0,20 | | | 0,14 | |
| | | 0,09 | | | 0,67 | | | 0,64 | |
| 3,0 | 5,73 | <0,01 | 0,15 | 1,21 | <0,01 | 0,69 | 1,02 | <0,01 | 0,65 |
| | | 0,06 | | | 0,02 | | | 0,01 | |
| | | 0,06 | | | 0,26 | | | 0,22 | |
| 3,5 | 1,92 | <0,01 | 0,06 | 0,58 | <0,01 | 0,26 | 0,49 | 0,01 | 0,23 |
| | | <0,01 | | | <0,01 | | | <0,01 | |
| | | 0,13 | | | 0,16 | | | 0,17 | |
| 4,0 | 0,72 | 0,02 | 0,15 | 0,50 | 0,04 | 0,20 | 0,45 | 0,04 | 0,21 |
| | | <0,01 | | | <0,01 | | | <0,01 | |
| | | 0,02 | | | 0,19 | | | 0,19 | |
| 4,5 | 1,01 | 0,14 | 0,34 | 0,77 | 0,09 | 0,29 | 0,66 | 0,09 | 0,28 |
| | | <0,01 | | | <0,01 | | | <0,01 | |
| | | 0,19 | | | 0,19 | | | 0,18 | |
| 5,0 | 0,78 | 0,09 | 0,28 | 0,75 | 0,09 | 0,28 | 0,76 | 0,09 | 0,27 |
| | | <0,01 | | | <0,01 | | | <0,01 | |
| | | 0,19 | | | 0,18 | | | 0,20 | |
| 5,5 | 0,77 | 0,10 | 0,29 | 0,72 | 0,10 | 0,28 | 0,99 | 0,13 | 0,33 |
| | | <0,10 | | | <0,01 | | | <0,01 | |
| | | 0,19 | | | 0,19 | | | 0,19 | |
| 6,0 | 0,78 | 0,11 | 0,30 | 0,75 | 0,11 | 0,30 | 0,86 | 0,12 | 0,31 |
| | | <0,01 | | | <0,01 | | | <0,01 | |

Die Ergebnisse der Experimente unter beschleunigten Bedingungen (Tabellen 2 und 3) erlaubten eine nähere Spezifizierung von Abbauprodukten und eine aufgeschlüsselte Relation der spezifischen Verunreinigungen bei dem jeweiligen pH-Wert. Damit lieferten diese Versuche eine Grundlage zur Wahl eines engeren pH-Intervalls.

Der Bereich des Stabilitätsoptimums von mit Phosphorsäure oder Schwefelsäure eingestellten Oxaliplatinlösungen mit übereinstimmender Stabilität mit den mit Oxalsäure eingestellten Lösungen liegt im Bereich von pH 3,5 bis 6,0; insbesondere im Bereich von pH 4,0 bis 6,0. Der relativ hohe Gehalt an Oxalsäure bei pH 3 bewirkt durch Produkthemmung eine noch weiter erhöhte Stabilität bei mit Oxalsäure eingestellten Lösungen. Doch sind Lösungen zur parenteralen Verabreichungen mit einem pH-Wert von ungefähr 3 wegen des damit verbundenen Injektionsschmerzes unerwünscht. Höhere pH-Werte (etwa pH 4 - 9) werden dagegen lokal allgemeinen besser vertragen und sind somit im allgemeinen vorzuziehen.

Bei pH-Werten unterhalb 3,0 bis 3,5 also bei saureren Lösungen, nimmt in allen Beispielen der Gehalt an unerwünschten unbekannten Verunreinigungen zu. Bei diesen pH-Werten wurde keine Bildung von Diaquo-DACH-Platin(II)-Dimer **(III)** aus Diaquo-DACH-Platin(II) (II) beobachtet. Während bei pH-Werten unterhalb von pH 3,5 im Fall der mit Phosphorsäure und mit Schwefelsäure eingestellten Lösungen ein deutlicher Anstieg von Diaquo-DACH-Platin(II) (II) beobachtet wird, wird im Fall der mit Oxalsäure eingestellten Lösungen bei diesen pH-Werten durch den deutlich höheren Gehalt an freier Oxalsäure in diesen Lösungen ein Anstieg der Konzentration des Diaquo-DACH-Platin(II) (**II**) unterdrückt. Als eine mögliche Ursache hierfür kann eine wirksame (Oxalsäure-) Produkthemmung durch eine Komplexrückbildungsreaktion aus Zersetzungsprodukten (Oxalsäure plus Diaquo-Dach-Platin(II) **(II)** zu Oxaliplatin) gemäß Massenwirkungsbeziehung des Oxaliplatin-Komplexgleichgewichts angenommen werden.

Ein Anstieg an unerwünschten unbekannten Verunreinigungen wird jedoch auch bei den mit Oxalsäure eingestellten Lösungen mit hohen Gehalten an Oxalsäure und stark sauren pH-Werten unterhalb von pH 3,0 nicht verhindert.

Im Ergebnis zeigen sich aus den vorgenannten Kinetiken (Tab. 2 und 3) als Stabilitätsoptimum folgende pH-Intervalle:
- bei mit Oxalsäure eingestellten Lösungen pH 3,0 - 6,0
- bei mit Phosphorsäure eingestellten Lösungen pH 3,5 - 6,0
- bei mit Schwefelsäure eingestellten Lösungen pH 3,5 - 6,0

Als Ergebnis zeigt sich damit in dieser Untersuchung, dass die Anionen der zu Oxalsäure alternativ gewählten Säuren zur Einstellung des pH-Wertes (Phosphorsäure und Schwefelsäure bzw. Phosphatanionen und Sulfatanionen) den Oxaliplatinkomplex nicht destabilisieren, wie es im Gegensatz dazu mit Zitronensäure (Citratanionen, pH 3, pH 5) und Essigsäure (Acetatanionen, pH 5) im europäischen Patent EP 0 943 331 dargestellt ist.

### Beispiel 3 - Vergleichsversuche unter Realbedingungen zur Stabilität von wässrigen Oxaliplatinlösungen unter Zusatz von Oxalsäure, Phosphorsäure bzw. Schwefelsäure im eingegrenzten pH-Bereich.

### Versuchsdesign

Die Herstellung der Versuchschargen erfolgte bei 15 - 20°C durch Lösen der entsprechenden Menge Oxaliplatin (C = 1 mg/ml bzw. C = 6,6 mg/ml), wobei als Lösungsmittel Wasser für Injektionsmittel diente, das zuvor mit q.s. 1N Oxalsäure, 1N Phosphorsäure bzw. 1N Schwefelsäure auf die Ziel-pH-Werte (3,5 - 4,0 - 5,0) voreingestellt wurde.

Die erhaltenen Testansätze wurden nach Durchführung der ersten Reinheitsanalysen sterilfiltriert, aseptisch in Durchstechflaschen abgefüllt, bei 2 - 8°C lichtgeschützt inkubiert und zu den vorgesehenen Prüftakten einer erneuten Analyse unterzogen. Die pH-Wertbestimmungen erfolgten in allen Messungen potentiometrisch.

Zum Vergleich der Qualität der erfindungsgemäßen Stabilisierung von Oxaliplatinlösungen durch andere Säuren als Oxalsäure, wurden die durch Zugabe von Phosphorsäure und Schwefelsäure erzielten Stabilitäten mit der verglichen, die durch einen gleichionigen Zusatz (Oxalsäure) erreicht wird (siehe Tabellen 4 und 5). Aus EP 0 943 331 war bekannt, dass durch Zusatz von Oxalsäure stabile, wässrige Oxaliplatinlösungen erhalten werden können. In den Versuchen zu vorliegender Erfindung wurden durch Oxalsäure folgende Stabilitäten erzielt.

**Tabelle 4: Stabilitäten von Oxaliplatin-Lösungen (C = 1 mg/ml) bei pH = 3,5, pH = 4 und pH = 5, wobei der pH-Wert durch 1N Oxalsäure eingestellt wurde.**

| Beispiel | pH-Wert | Prüftakt Bei 2 - 8°C | Diaquo-DACH-Platin **II** | Diaquo-DACH-Platin Dimer **III** | Unbekannte Verunreinigungen |
|---|---|---|---|---|---|
| | | | (% w/w) | (% w/w) | (% w/w) |
| pH 3,5 | 3,48 | Start | 0,14 | <0,06 | 0 |
| | 3,48 | 1 mon | 0,14 | <0,06 | <0,06 |
| | 3,52 | 3 mon | 0,13 | <0,06 | <0,06 |
| | 3,50 | 12 mon | 0,12 | <0,06 | <0,06 |
| pH 4 | 3,89 | Start | 0,13 | <0,06 | 0 |
| | 3,99 | 1 mon | 0,12 | <0,06 | <0,06 |
| | 4,07 | 3 mon | 0,13 | <0,06 | <0,06 |
| | 4,00 | 12 mon | 0,13 | <0,06 | <0,06 |
| pH 5 | 4,93 | Start | 0,19 | 0,15 | 0 |
| | 4,99 | 1 mon | 0,22 | 0,17 | <0,06 |
| | 5,15 | 3 mon | 0,27 | 0,19 | 0,07 |
| | 5,13 | 12 mon | 0,31 | 0,25 | 0,08 |

**Tabelle 5:Stabilitäten von Oxaliplatin-Lösungen (C = 6,6 mg/ml) bei pH = 3,5, pH = 4 und pH = 5, wobei der pH-Wert durch 1N Oxalsäure eingestellt wurde.**

| Beispiel | pH-Wert | Prüftakt bei 2 - 8°C | Diaquo-DACH-Platin **II** | Diaquo-DACH-Platin Dimer **III** | Unbekannte Verunreinigungen |
|---|---|---|---|---|---|
| | | | (% w/w) | (% w/w) | (% w/w) |
| pH 3,5 | 3,44 | Start | 0,14 | <0,01 | 0 |
| | 3,48 | 1 mon | 0,14 | <0,01 | <0,01 |
| | 3,54 | 3 mon | 0,14 | <0,01 | 0,01 |
| | 3,50 | 12 mon | 0,13 | <0,01 | 0,02 |
| pH 4 | 3,81 | Start | 0,14 | 0,03 | 0 |
| | 3,92 | 1 mon | 0,13 | <0,01 | 0,01 |
| | 4,02 | 3 mon | 0,12 | <0,01 | 0,02 |
| | 4,01 | 12 mon | 0,14 | <0,01 | 0,03 |
| pH 5 | 4,93 | Start | 0,17 | 0,16 | 0 |
| | 4,99 | 1 mon | 0,21 | 0,15 | 0,04 |
| | 5,15 | 3 mon | 0,26 | 0,18 | 0,07 |
| | 5,03 | 12 mon | 0,30 | 0,23 | 0,09 |

Unter identischen Bedingungen wurden Untersuchungen an erfindungsgemäßen, durch den Zusatz von Phosphorsäure stabilisierten Oxaliplatinlösungen durchgeführt. In diesen Versuchen wurden folgende Stabilitäten erzielt (Tabellen 6 und 7).

**Tabelle 6:Stabilitäten von Oxaliplatin-Lösungen (C = 1,0 mg/ml) bei pH = 3,5, pH = 4 und pH = 5, wobei der pH-Wert durch 1N Phosphorsäure eingestellt wurde.**

| Beispiel | pH-Wert | Prüftakt bei 2 - 8°C | Diaquo-DACH-Platin **II** | Diaquo-DACH-Platin Dimer **III** | Unbekannte Verunreinigungen |
|---|---|---|---|---|---|
| | | | (% w/w) | (% w/w) | (% w/w) |
| pH 3,5 | 3,44 | Start | 0,12 | <0,06 | 0 |
| | 3,48 | 1 mon | 0,13 | <0,06 | <0,06 |
| | 3,47 | 3 mon | 0,13 | <0,06 | <0,06 |
| | 3,43 | 12 mon | 0,14 | <0,06 | <0,06 |
| pH 4 | 3,92 | Start | 0,13 | <0,06 | 0 |
| | 3,93 | 1 mon | 0,12 | <0,06 | <0,06 |
| | 4,03 | 3 mon | 0,12 | <0,06 | <0,06 |
| | 4,05 | 12 mon | 0,13 | <0,06 | <0,06 |
| pH 5 | 5,02 | Start | 0,18 | 0,14 | 0 |
| | 4,92 | 1 mon | 0,21 | 0,18 | <0,06 |
| | 5,12 | 3 mon | 0,26 | 0,20 | 0,07 |
| | 5,10 | 12 mon | 0,30 | 0,23 | 0,06 |

**Tabelle 7:Stabilitäten von Oxaliplatin-Lösungen (C = 6,6 mg/ml) bei pH = 3,5, pH = 4 und pH = 5, wobei der pH-Wert durch 1N Phosphorsäure eingestellt wurde.**

| Beispiel | pH-Wert | Prüftakt bei 2 - 8° C | Diaquo-DACH-Platin **II** | Diaquo-DACH-Platin Dimer **III** | Unbekannte Verunreinigungen |
|---|---|---|---|---|---|
| | | | (% w/w) | (% w/w) | (% w/w) |
| pH 3,5 | 3,42 | Start | 0,14 | <0,01 | 0 |
| | 3,44 | 1 mon | 0,13 | <0,01 | <0,01 |
| | 3,44 | 3 mon | 0,13 | <0,01 | 0,01 |
| | 3,46 | 12 mon | 0,14 | <0,01 | 0,02 |
| pH 4 | 4,08 | Start | 0,14 | 0,01 | 0 |
| | 4,00 | 1 mon | 0,14 | <0,01 | 0,01 |
| | 4,02 | 3 mon | 0,13 | <0,01 | 0,02 |
| | 4,08 | 12 mon | 0,14 | <0,01 | 0,02 |
| pH 5 | 5,02 | Start | 0,17 | 0,14 | 0 |
| | 5,08 | 1 mon | 0,22 | 0,13 | 0,03 |
| | 5,19 | 3 mon | 0,25 | 0,17 | 0,07 |
| | 5,05 | 12 mon | 0,28 | 0,21 | 0,10 |

Wiederum unter identischen Bedingungen wurden Untersuchungen an Schwefelsäurestabilisierten Oxaliplatinlösungen durchgeführt. In diesen Versuchen zu vorliegender Erfindung wurden durch Schwefelsäure folgende Stabilitäten erzielt (Tabellen 8 und 9).

**Tabelle 8:Stabilitäten von Oxaliplatin-Lösungen (C = 1,0 mg/ml) bei pH = 3,5, pH = 4 und pH = 5, wobei der pH-Wert durch 1N Schwefelsäure eingestellt wurde.**

| Beispiel | pH-Wert | Prüftakt bei 2 - 8°C | Diaquo-DACH-Platin **II** | Diaquo-DACH-Platin Dimer **III** | Unbekannte Verunreinigungen |
|---|---|---|---|---|---|
| | | | (% w/w) | (% w/w) | (% w/w) |
| pH 3,5 | 3,56 | Start | 0,14 | <0,06 | 0 |
| | 3,54 | 1 mon | 0,13 | <0,06 | <0,06 |
| | 3,53 | 3 mon | 0,13 | <0,06 | <0,06 |
| | 3,52 | 12 mon | 0,14 | <0,06 | <0,06 |
| pH 4 | 4,08 | Start | 0,14 | <0,06 | 0 |
| | 4,02 | 1 mon | 0,13 | <0,06 | <0,06 |
| | 4,10 | 3 mon | 0,12 | <0,06 | <0,06 |
| | 4,11 | 12 mon | 0,14 | <0,06 | <0,06 |
| pH 5 | 4,96 | Start | 0,19 | 0,13 | 0 |
| | 4,98 | 1 mon | 0,22 | 0,18 | <0,06 |
| | 5,03 | 3 mon | 0,25 | 0,19 | 0,07 |
| | 5,11 | 12 mon | 0,31 | 0,24 | 0,08 |

**Tabelle 9:Stabilitäten von Oxaliplatin-Lösungen (C = 6,6 mg/ml) bei pH = 3,5, pH = 4 und pH = 5, wobei der pH-Wert durch 1N Schwefelsäure eingestellt wurde.**

| Beispiel | pH-Wert | Prüftakt bei 2 - 8°C | Diaquo-DACH-Platin **II** | Diaquo-DACH-Platin Dimer **III** | Unbekannte Verunreinigungen |
|---|---|---|---|---|---|
| | | | (% w/w) | (% w/w) | (% w/w) |
| pH 3,5 | 3,53 | Start | 0,14 | <0,01 | 0 |
| | 3,54 | 1 mon | 0,14 | <0,01 | <0,01 |
| | 3,56 | 3 mon | 0,13 | <0,01 | 0,01 |
| | 3,52 | 12 mon | 0,13 | <0,01 | 0,02 |
| pH 4 | 4,08 | Start | 0,14 | 0,01 | 0 |
| | 4,01 | 1 mon | 0,14 | <0,01 | 0,01 |
| | 4,03 | 3 mon | 0,13 | <0,01 | 0,02 |
| | 4,09 | 12 mon | 0,15 | <0,01 | 0,02 |
| pH 5 | 5,08 | Start | 0,18 | 0,14 | 0 |
| | 5,01 | 1 mon | 0,23 | 0,13 | 0,03 |
| | 4,99 | 3 mon | 0,25 | 0,18 | 0,05 |
| | 5,05 | 12 mon | 0,29 | 0,20 | 0,08 |

Ein Vergleich der erfindungsgemäßen Lösungen unter Zusatz von Phosphorsäure oder Schwefelsäure mit Oxaliplatinlösungen ohne Säurezusatz (Beispiel 1) bzw. mit Oxalsäure-Zusatz gemäß dem Stand der Technik zeigt, dass zum einen die pH-Werte der gelagerten vorgenannten Lösungen über die Dauer der Stabilitätsuntersuchung bei 2 - 8°C mit nur geringen Abweichungen von +/- 0,2 pH-Stufen unverändert blieben.

Zum anderen zeigte sich überraschend, dass Oxaliplatinlösungen, deren pH-Wert auf einen Ziel-pH-Wert von 3,5 bis 5,0 mit Phosphorsäure oder Schwefelsäure eingestellt wurde, genauso stabil sind wie vorbekannte Oxaliplatinlösungen, deren pH-Wert mit Oxalsäure ebenfalls auf einen Wert von 3,5 bis 5,0 eingestellt wurde.

Gleichfalls und ebenso überraschend zeigte sich in diesen Experimenten eine Übereinstimmung hinsichtlich der pH-Abhängigkeit der Zunahme der Summe der Verunreinigungen im Vergleich zu vorbekannten Oxaliplatinlösungen, deren pH-Wert mit Oxalsäure eingestellt wurde. Mit den beschleunigten Stabilitätstests hat sich erwiesen, dass für eine Stabilisierung des Oxaliplatinkomplexes in wässriger Lösung auch andere als gleichionige Säuren (Oxalsäure) und deren Anionen (Oxalate) geeignet sind. Erst bei sehr niedrigen pH-Werten, die für eine parenterale Verabreichung praktisch ungeeignet sind, kommen im Fall der oxalsäurestabilisierten Lösungen die Effekte der Produkthemmung und der Komplexrückbildung, die durch die hohe Oxalsäurekonzentration in diesen Fällen hervorgerufen werden, zum Tragen. Weiterhin hat sich in den durchgeführten Versuchen gezeigt, dass Phosphorsäure und Schwefelsäure keine reaktiven Anionen erzeugen, die den Oxaliplatinkomplex in wässriger Lösung destabilisieren.

Es hat sich insbesondere gezeigt, dass vorbekannte Oxaliplatinlösungen, deren pH-Wert auf Werte im Bereich von ca. 3,5 bis ca. 5,0 mit Oxalsäure eingestellt wurde, in keiner Weise stabiler sind als die erfindungsgemäßen Oxaliplatin-Lösungen, die beispielhaft durch Phosphor- bzw. Schwefelsäure auf einen gleichermaßen sauren pH-Wert eingestellt wurden. In diesen Experimenten hat sich völlig überraschend gezeigt, dass die erfindungsgemäßen Lösungen wenigstens so stabil sind wie solche, die durch Oxalsäure stabilisiert sind, ohne deren oben genannten negativen Wirkungen zu haben.

### Beispiel 4 - Konzentrationsabhängigkeit der Stabilität

Es wurden Oxaliplatin-haltige Lösungen mit Konzentrationen von 0,025, 0,25, 1,0, 5,0 und 6,6 mg/ml in Wasser hergestellt. In 80% des zur Herstellung der Lösungen erforderlichen und auf 35-45°C erwärmten Wassers für Injektionen wurde der jeweilige pH-Wert mit q.s. 1N Phosphorsäure auf pH = 3 (+/-0,2), bzw. pH = 4 (+/-0,2) bzw. pH = 5 (+/-0,2) eingestellt, die entsprechende Menge Oxaliplatin gelöst, und der Ansatz auf Raumtemperatur abgekühlt. Die Ansätze wurden mit Wasser für Injektionen aufgefüllt und sterilfiltriert. Schließlich wurden sie in 5ml Durchstechflaschen aseptisch abgefüllt, und diese mit Injektionsstopfen und Bördelkappen luftdicht verschlossen.

Die Chargen wurden lichtgeschützt über eine Dauer von 12 Monaten bei 2 - 8°C gelagert. Die Gehaltsbestimmung erfolgte mittels HPLC und die pH-Bestimmung erfolgte potentiometrisch. Die erhaltenen Ergebnisse sind in der Tabelle 10 zusammengefasst.

**Tabelle 10:Konzentrationsabhängigkeit der Stabilität von Oxaliplatin-Lösungen. pH-Werte eingestellt mit q.s. Phosphorsäure (12 mon, 2 - 8°C).**

| Konzentration | pH 3 | pH 4 | pH 5 |
|---|---|---|---|
| 0,025 mg/ml | 99,4% | 99,2% | 98,4% |
| 0,25 mg/ml | 99,8% | 99,6% | 98,7% |
| 1,0 mg/ml | 100,3% | 99,3% | 99,0% |
| 5,0 mg/ml | 99,9% | 99,1% | 98,6% |
| 6,6 mg/ml | 99,8% | 99,2% | 98,4% |

Wie deutlich aus der Tabelle 11 zu erkennen ist, werden die erfindungsgemäßen Lösungen selbst bei Konzentrationen bis hinunter zu C = 0,025 mg/ml durch den Zusatz von Phosphorsäure stabilisiert.

### Beispiele 5 - 99 - Zusammensetzung exemplarischer, erfindungsgemäßer, gebrauchsfertiger Oxaliplatinlösungen

Im folgenden sind bevorzugte Ausführungsformen der erfindungsgemäßen Oxaliplatinlösungen exemplifiziert.

**Tabelle 11:Bevorzugte erfindungsgemäße Oxaliplatinlösungen**

| No | Oxaliplatin (mg/ml) | pH-Wert | Säure | Kohlenhydrat, Hydroxyderivat | Pufferbase |
|---|---|---|---|---|---|
| 5. | 0,10 | 3,0 | Phosphorsäure | 10 mg/ml Glucose Monohydrat, | 5 mg/ml Dinatrium- |
| | | | | 100 mg/ml Polyethylenglycol 400 | hydrogenphosphat |
| 6. | 0,25 | 5,8 | Phosphorsäure | 10 mg/ml Glucose Monohydrat, | 20 mg/ml Dinatrium- |
| | | | | 200 mg/ml Polyethylenglycol 400 | hydrogenphosphat |
| 7. | 0,25 | 3,8 | Phosphorsäure | 35 mg/ml Glucose Monohydrat, | 20 mg/ml Dinatrium- |
| | | | | 10 mg/ml Polyethylenglycol 400 | hydrogenphosphat |
| 8. | 0,25 | 4,8 | Phosphorsäure | 35 mg/ml Glucose Monohydrat | 20 mg/ml Dinatriumhydrogenphosphat |
| 9. | 0,25 | 4,8 | Phosphorsäure | 35 mg/ml Glucose Monohydrat | 20 mg/ml Dinatriumhydrogenphosphat |
| 10. | 0,25 | 3,8 | Phosphorsäure | 35 mg/ml Glucose Monohydrat | 0,2 mg/ml Dinatriumhydrogenphosphat |
| 11. | 0,25 | 3,4 | Phosphorsäure | 55 mg/ml Glucose Monohydrat | 20 mg/ml Natriumlactat |
| 12. | 0,25 | 5,8 | Phosphorsäure | 75 mg/ml Glucose Monohydrat | |
| 13. | 0,25 | 4,8 | Phosphorsäure | | |
| 14. | 0,25 | 3,8 | Schwefelsäure | 40 mg/ml Glucose Monohydrat | 0,15 mg/ml Dinatriumhydrogenphosphat |
| 15. | 0,25 | 5,8 | Schwefelsäure | 40 mg/ml Glucose Monohydrat | |
| 16. | 0,25 | 5,8 | Schwefelsäure | | |
| 17. | 0,5 | 3,2 | Schwefelsäure | 40 mg/ml Glucose Monohydrat | |
| 18. | 0,5 | 3,2 | Phosphorsäure | 40 mg/ml Glucose Monohydrat | |
| 19. | 0,5 | 3,2 | Phosphorsäure | | |
| 20. | 1,0 | 3,6 | Phosphorsäure | 60 mg/ml Glucose Monohydrat | 0,10 mg/ml Dinatriumhydrogenphosphat |
| 21. | 1,0 | 3,6 | Phosphorsäure | | |
| 22. | 1,0 | 3,6 | Schwefelsäure | | |
| 23. | 2,0 | 3,6 | Phosphorsäure | | 0,5 mg/ml Dinatriumhydrogenphosphat |
| 24. | 2,0 | 3,6 | Phosphorsäure | | |
| 25. | 3,0 | 4,0 | Schwefelsäure | 50 mg/ml Glucose Monohydrat | |
| 26. | 3,0 | 3,0 | Schwefelsäure | 50 mg/ml Glucose Monohydrat | |
| 27. | 3,0 | 3,0 | Phosphorsäure | | |
| 28. | 5,0 | 5,9 | Phosphorsäure | | |
| 29. | 5,0 | 4,7 | Phosphorsäure | | 0,5 mg/ml Dinatriumhydrogenphosphat |
| 30. | 5,0 | 3,0 | Phosphorsäure | | |
| 31. | 5,0 | 3,5 | Phosphorsäure | | 5 mg/ml Dinatriumhydrogenphosphat |
| 32. | 5,0 | 4,0 | Schwefelsäure | 100 mg/ml Polyethylenglycol 600 | |
| 33. | 5,0 | 3,0 | Phosphorsäure | 55 mg/ml Glucose Monohydrat | 0,5 mg/ml Dinatriumhydrogenphosphat |
| 34. | 5,0 | 3,0 | Schwefelsäure | | |
| 35. | 5,0 | 3,0 | Schwefelsäure | | |
| 36. | 6,0 | 3,5 | Phosphorsäure | 55 mg/ml Glucose Monohydrat | 0,5 mg/ml Dinatriumhydrogenphosphat |
| 37. | 6,0 | 3,5 | Phosphorsäure | 55 mg/ml Glucose Monohydrat | |
| 38. | 6,0 | 3,5 | Phosphorsäure | | |
| 39. | 6,0 | 3,8 | Phosphorsäure | | |
| 40. | 6,0 | 3,2 | Phosphorsäure | | |
| 41. | 6,0 | 3,0 | Phosphorsäure | | |
| 42. | 6,0 | 4,8 | Phosphorsäure | | |
| 43. | 6,0 | 3,0 | Phosphorsäure | 55 mg/ml Glucose Monohydrat | 2,0 mg/ml Natriumlactat |
| 44. | 6,0 | 3,0 | Phosphorsäure | 55 mg/ml Glucose Monohydrat | 0,15 mg/ml Dinatriumhydrogenphosphat |
| 45. | 6,0 | 3,5 | Phosphorsäure | | 0,15 mg/ml Dinatriumhydrogenphosphat |
| 46. | 6,0 | 3,5 | Phosphorsäure | 55 mg/ml Glucose Monohydrat | |
| 47. | 6,0 | 3,5 | Phosphorsäure | 55 mg/ml Glucose Monohydrat | 2,0 mg/ml Natriumlactat |
| 48. | 6,0 | 3,5 | Phosphorsäure | 55 mg/ml Glucose Monohydrat | 6,0 mg/ml Natriumlactat |
| 49. | 6,0 | 3,5 | Phosphorsäure | 55 mg/ml Glucose Monohydrat | 0,35 mg/ml Dinatriumhydrogenphosphat |
| 50. | 6,0 | 4,0 | Phosphorsäure | | |
| 51. | 6,0 | 4,0 | Phosphorsäure | | 4,0 mg/ml Natriumlactat |
| 52. | 6,0 | 3,5 | Phosphorsäure | 50 mg/ml Glucose Monohydrat | 5 mg/ml Dinatriumhydrogenphosphat |
| 53. | 6,0 | 3,0 | Phosphorsäure | 80 mg/ml Glucose Monohydrat | 15 mg/ml Dinatriumhydrogenphosphat |
| 54. | 6,0 | 3,0 | Phosphorsäure | 80 mg/ml Glucose Monohydrat | 1 mg/ml Dinatriumhydrogenphosphat |
| 55. | 6,0 | 4,0 | Phosphorsäure | 80 mg/ml Glucose Monohydrat | 15 mg/ml Dinatriumhydrogenphosphat |
| 56. | 6,0 | 3,5 | Schwefelsäure | 65 mg/ml Glucose Monohydrat | |
| 57. | 6,0 | 3,5 | Schwefelsäure | 10 mg/ml Glucose Monohydrat | 0,25 mg/ml Dinatriumhydrogenphosphat |
| 58. | 6,0 | 3,5 | Schwefelsäure | | |
| 59. | 6,0 | 3,5 | Schwefelsäure | | |
| 60. | 6,0 | 3,5 | Schwefelsäure | 45 mg/ml Glucose Monohydrat | |
| 61. | 6,0 | 5,8 | Schwefelsäure | 35 mg/ml Glucose Monohydrat | 0,55 mg/ml Dinatriumhydrogenphosphat |
| 62. | 6,0 | 3,0 | Schwefelsäure | 35 mg/ml Glucose Monohydrat | 1,5 mg/ml Dinatriumhydrogenphosphat |
| 63. | 6,0 | 3,4 | Schwefelsäure | 30 mg/ml Glucose Monohydrat | 2,5 mg/ml Dinatriumhydrogenphosphat |
| 64. | 6,0 | 3,8 | Schwefelsäure | 50 mg/ml Glucose Monohydrat | 0,15 mg/ml Dinatriumhydrogenphosphat |
| 65. | 6,0 | 5,8 | Schwefelsäure | 5 mg/ml Glucose Monohydrat | |
| 66. | 6,0 | 3,0 | Schwefelsäure | 35 mg/ml Glucose Monohydrat | |
| 67. | 6,0 | 3,4 | Schwefelsäure | 35 mg/ml Glucose Monohydrat | |
| 68. | 6,0 | 3,8 | Schwefelsäure | 35 mg/ml Glucose Monohydrat | |
| 69. | 6,0 | 3,0 | Schwefelsäure | | |
| 70. | 6,0 | 3,4 | Schwefelsäure | | |
| 71. | 6,0 | 3,8 | Schwefelsäure | | |
| 72. | 7,0 | 3,8 | Phosphorsäure | 100 mg/ml Glucose Monohydrat | 0,25 mg/ml Dinatriumhydrogenphosphat |
| 73. | 7,0 | 3,4 | Phosphorsäure | 100 mg/ml Glucose Monohydrat | |
| 74. | 7,0 | 3,4 | Phosphorsäure | 200 mg/ml Glucose Monohydrat | |
| 75. | 7,0 | 4,4 | Schwefelsäure | 100 mg/ml Glucose Monohydrat | |

## Patentansprüche

1. Pharmazeutische Zusammensetzung, die Wasser, Oxaliplatin und eine anorganische Säure umfasst, wobei das Anion der Säure die Stabilität der Lösung nicht beeinträchtigt, wobei die Säure ausgewählt ist aus der Gruppe Phosphorsäure, Schwefelsäure oder Mischungen davon, und wobei der pH-Wert auf einen Wert innerhalb des Bereichs zwischen 3 und 6 eingestellt wurde.

2. Pharmazeutische Zusammensetzung nach Anspruch 1, die gegebenenfalls zusätzlich weitere tonisierende, den pH-Wert einstellende, puffernde oder konservierende Hilfsstoffe umfasst.

3. Pharmazeutische Zusammensetzung nach einem der Ansprüche 1 oder 2, wobei der pH-Wert auf einen Wert innerhalb des Bereichs zwischen 3,5 und 5,0 eingestellt wurde.

4. Pharmazeutische Zusammensetzung nach einem der Ansprüche 1 bis 3, wobei die Oxaliplatin-Konzentration zwischen 0,025 mg/ml und 25 mg/ml liegt.

5. Pharmazeutische Zusammensetzung nach einem der Ansprüche 1 bis 4, die nicht unmittelbar vor der Verabreichung an einen Patienten aus einer festen Oxaliplatin-Zubereitung rekonstituiert wurde.

6. Verwendung der pharmazeutischen Zusammensetzung nach einem der Ansprüche 1 bis 5 zur Herstellung eines Arzneimittels zur Behandlung einer Tumorerkrankung.

7. Verwendung einer oder mehrerer anorganischen Säure, deren Anion die Stabilität der Lösung nicht beeinträchtigt, zur Herstellung einer stabilen, pharmazeutisch akzeptablen Lösung von Oxaliplatin in Wasser, wobei die Säure ausgewählt ist aus der Gruppe Phosphorsäure, Schwefelsäure oder Mischungen davon, und wobei der pH-Wert auf einen Wert innerhalb des Bereichs zwischen 3 und 6 eingestellt wurde.

8. Verwendung einer oder mehrerer anorganischen Säure zur Stabilisierung von pharmazeutisch akzeptablen Lösungen von Oxaliplatin in Wasser, wobei die Säure ausgewählt ist aus der Gruppe Phosphorsäure, Schwefelsäure oder Mischungen davon, und wobei der pH-Wert auf einen Wert innerhalb des Bereichs zwischen 3 und 6 eingestellt wurde.

## Claims

1. Pharmaceutical composition comprising water, oxaliplatin, and an inorganic acid, wherein the anion of the acid does not impair the stability of the solution, whereby the acid is selected from the group consisting of phosphoric acid, sulphoric acid, and mixtures thereof, and whereby the pH-value is adjusted to a value between 3 and 6.

2. Pharmaceutical composition according to claim 1 comprising, if required, additional tonic, pH-value adjusting, buffer, or conserving excipients.

3. Pharmaceutical composition according to claim 1 or claim 2, wherein the pH-value is adjusted to a value in the area between 3,5 and 5,0.

4. Pharmaceutical composition according to one of the claims 1 - 3, whereby the oxaliplatin-concentration is in between 0,025 mg/ml and 25 mg/ml.

5. Pharmaceutical composition according to one of claims 1 - 4 that has not been reconstituted from a solid oxaliplatin-composition directly before administration to a patient.

6. Utilization of the pharmaceutical composition according to anyone of claims 1-5 for the manufacturing of a medicament for the treatment of tumor diseases.

7. Utilization of one or several organic acids, the anion of which does not impair the stability of the solution, for the preparation of a stable, pharmaceutically acceptable solution of oxaliplatin in water, wherein the acid is chosen from the group consisting of phosphoric acid, sulphoric acid, and mixtures thereof, and whereby the pH-value is adjusted to a value in the area between 3 and 6.

8. Utilization of one or several inorganic acids for the stabilization of pharmaceutically acceptable solutions of oxaliplatin in water, whereby the acid is chosen from the group consisting of phosphoric acid, sulphoric acid, and mixtures thereof, and whereby the pH-value is adjusted to a value in the area between 3 and 6.

## Revendications

1. Composition pharmaceutique contenant de l'eau, de l'oxaliplatine et un acide inorganique, l'anion de l'acide n'affectant pas la stabilité de la solution, l'acide étant choisi dans le groupe formé par l'acide phosphorique, l'acide sulfurique ou des mélanges de ceux-ci, et le pH étant établi à une valeur comprise entre 3 et 6.

2. Composition pharmaceutique selon la revendication 1, comprenant éventuellement, à titre complémentaire, d'autres adjuvants ayant une action de tonisation, d'ajustement du pH, de tamponnement ou de conservation.

3. Composition pharmaceutique selon l'une quelconque des revendications 1 ou 2, dont le pH est établi à une valeur comprise entre 3, 5 et 5,0.

4. Composition pharmaceutique selon l'une quelconque des revendications 1 à 3, dans laquelle la concentration d'oxaliplatine est comprise entre 0,025 mg/ml et 25 mg/ml.

5. Composition pharmaceutique selon l'une quelconque des revendications 1 à 4, qui n'est pas reconstituée immédiatement avant l'administration à un patient à partir d'une préparation solide d'oxaliplatine.

6. Utilisation de la composition pharmaceutique selon l'une quelconque des revendications 1 à 5 pour préparer un médicament destiné à traiter les tumeurs.

7. Utilisation d'un ou plusieurs acides inorganiques, dont l'anion n'affecte pas la stabilité de la solution, pour préparer une solution stable et pharmacologiquement acceptable d'oxaliplatine dans l'eau, l'acide étant choisi parmi le groupe formé par l'acide phosphorique, l'acide sulfurique ou des mélanges de ceux-ci, et le pH étant établi à une valeur comprise entre 3 et 6.

8. Utilisation d'un ou plusieurs acides inorganiques pour stabiliser des solutions pharmacologiquement acceptables d'oxaliplatine dans l'eau, l'acide étant choisi parmi le groupe formé par l'acide phosphorique, l'acide sulfurique ou des mélanges de ceux-ci, et le pH étant établi à une valeur comprise entre 3 et 6.
